# EUROPEAN PATENT SPECIFICATION

(11) **EP 1 785 165 B1**
(45) Date of publication and mention of the grant of the patent: **01.12.2010**
(21) Application number: 05742397.2
(22) Date of filing: 26.04.2005
(51) Int. Cl.: A62B 23/06

(54) **Anatomical and medical nasal respirator**
Anatomische und medizinische Nasenatmungsvorrichtung
Respirateur anatomique et medicale nasal

(30) Priority: 26.04.2004 ES 200400992
(43) Date of publication of application: 16.05.2007
(73) Proprietor: Guildpharma, S.L., 03440 IBI (Alicante) (ES)
(72) Inventor: Guildpharma, S.L., 03440 IBI (Alicante) (ES)
(74) Representative: Carpintero Lopez, Francisco
(86) International application number: PCT/ES2005/000220
(87) International publication number: WO 2005/113072

(56) References cited:
- EP-A2- 1 340 522
- GB-A- 138 079
- US-A- 2 151 227

## Description

### OBJECT OF THE INVENTION

This invention relates to a new type of anatomical inhaler, which when introduced into the nasal cavities, facilitates breathing for people who for different reasons have said cavities totally or partially obstructed.

### BACKGROUND OF THE INVENTION

We have all had occasion, at some point in our lives, to experiment the discomfort of a stuffed nose. Doubtless there exists an instinctive desire to breathe through one's nose. Attempting, when one's nose is blocked, to overcome the resistance put up by the nasal passages against the movement of air is associated with a feeling of general discomfort and tiredness. The normal functioning of the entire respiratory system depends on adequate nasal permeability.

In a very general way, we could define nasal obstruction as a situation in which a sufficient quantity of air does not reach the lungs upon breathing exclusively through one's nose.

In new-borns and lactating infants, a nasal blockage, even if temporary, can create a considerable concern due to its severity, since infants are nasal breathers that do not know how to breathe through their mouths, and in addition they need to have an unblocked nose in order to be fed correctly. That is, in order to suckle the mother's breast or a bottle, a sufficient passage of air is required by way of the nose. Bigger children are better able to tolerate nasal obstruction, although if it is chronic it can cause malformation in their mouths and face. Normally this nasal obstruction is brought about by large or inflamed adenoid vegetation due to a nasal infection, to foreign bodies that children often put in their noses, but it can also be provoked by birth defects such as the lack of perforation of the posterior nasal orifice or choanal atresia, tumours, traumatism or many other causes. On many occasions large and inflamed adenoid pharyngeal polypus are accompanied by inflammation of the mucous of the middle ear, provoking the accumulation of serosity and mucosity in the tympanum, which is known by the name of otitis secretory or mucous-secretory otitis. Frequently, such conditions are not painful, although they always bring about a more or less severe degree of hypoacusia or hearing loss. Such cases, very often, must be treated surgically, undertaking the removal, under general anaesthesia, of the pharyngeal polypus together with a small incision made, under the control of the microscope, in the tympanum membrane, in order to suck up the mucous from the middle ear and insert a ventilation tube. Especially in children, any obstruction of the nasal cavities must be studied and treated correctly in order to avoid serious complications, in very small children, and mouth and face deformation, in older children.

In adults, nasal obstruction can be due, also, to many causes. Infections caused by a virus or bacteria, especially sinusitis, both benign and malign tumours, traumatism, nose malformations, metabolic disorders, allergies, etc. should all be mentioned.

In a practical way, it can be summed up that a nasal obstruction is either due to a disorder in the functioning of erectile structures, located within the nasal cavities and called nasal concha, within which should be considered inflammations of nasal mucous provoked by allergies, medication, irritating substances, infections, etc., or to a disorder of the anatomical structures of the nose, both within (nasal cavity and nasal wall), and without (nasal cone).

The treatment of nasal obstructions provoked by a functional disorder will be medical and vary according to the cause that has provoked it; likewise, when nasal obstruction is provoked by an anatomical disorder of the nasal structures, the treatment will be surgical in the majority of cases. The type of surgical intervention will obviously also depend on the cause that provokes it.

Although it is true that there is a great number of nasal obstructions that do not require any treatment since they heal spontaneously, it should be emphasised that a doctor should be consulted regarding any persistent nasal obstruction, since it can be either a symptom of a banal cause, or the manifestation of a severe illness that requires early medical or surgical treatment.

For cases of temporary nasal obstruction where medical treatment is not immediately required, there are nasal strips that consist of a kind of gauze made up of two plastic filaments which, upon being folded over the nasal bone, create resistance by way of which the nostril wings are opened.

According to studies carried out at the Department of Otorhinolaryngology of the University of Ulleval, in Oslo (Norway), Dr. Djupesland has demonstrated that these nasal strips can "provide benefits to persons who suffer from slight snoring and objective nasal obstruction, caused by seasonal allergies or rhinitis, although those who suffer severe apneas or have a very large nose will probably not notice the difference if they use the dilator". Likewise, pregnant women in the final months of gestation can also benefit from dilators.

The Norwegian specialist explains that his double blind study, with placebo, included 20 patients with objective nasal obstruction of an average of 35% throughout the entire night.

The subjective evaluation of the usefulness of the nasal strips implied a significant improvement in sleep quality; reduction of mouth dryness and greater sensation of a clear nose. The objective measurements taken by way of acoustic revealed an increase in the size of the nostril passages that persisted throughout the night.

Another noteworthy result of the study was that the beneficial action of the nasal strips increased when the patients observed took up a lateral sleeping posture, in contrast to a supine one.

Other studies have questioned the usefulness of nasal strips, such as those carried out by the University of Buffalo (U.S.A.), coordinated by Dr. Frank Cerny. In this study it is demonstrated that the use of strips in athletes, improved air capture is not produced, nor, hence, is their performance improved.

In this sense, Dr. Djupesland has affirmed that the usefulness of dilators is just one factor among many others that are combined in the remission of snoring, such as a careful diet, not being overweight, tobacco or alcohol, especially before going to sleep. All in all, the otorhino-laryngologist has affirmed that this method is palliative for relief of slight snoring, and should not be considered to be a solution for more severe problems such as apnea.

Document EP1340522 A2 discloses a nasal filtration device for removing minute particles from the air entering the user's nostrils, the device comprising: a nasal insert adapted for insertion into a nostril of the user, the insert comprising a housing having an interior surface defining a passageway therethrough; and a filter element disposed within the housing and extending across the passageway, wherein the filter element is effective to prevent passage of particles greater than 1 micron in size. This filtration device is intended to filter the air flow entering into the user's nostrils, therefore making harder to breath, the opposite aim of the present invention which is to make it easier.

With the objective of solving the problems described above, a new type of mechanism has been developed that substantially improves breathing in cases in which the affected person should have an anatomical, morphological and physiological condition of the nose itself, smoking habits, winter pathologies, etc. that may bring about a ventilation insufficiency of the nasal cavities. This implicates compensating the respiratory function by breathing through the mouth, motivating disorders such as mouth dryness, night snoring, sleep disorders, etc.

On the other hand, limitations in physical exercise and sporting activity, in general, can be impairment caused by respiratory insufficiency through the nasal passages.

### DESCRIPTION OF THE INVENTION

The inhaler of the invention is made up of two empty cylinders, with a completely smooth interior surface, generally parallel and joined by a linking bridge, and two terminals, one for each cylinder.

At the end of each cylinder corresponding to the part that enters more deeply into the nasal passage, the cylinder is threaded on the exterior; the length of the threaded zone is less than 5 mm, preferably of 3 mm.

On each aforementioned end area of each cylinder, a cylindrical terminal is screwed on and is threaded on its interior, which may have different lengths, although there are two standard lengths: a shorter length of 5 mm, and a longer one of 9 mm, although they can be made with lengths varying by intervals of 2 mm.

The mounted assembly formed by each cylinder and its corresponding threaded terminal may present three peripheral rings, one external and the other intermediate, located on each cylinder, and one internal (located at the threaded terminal, on the part of the inhaler that it inserted more deeply into the nasal cavity) with a semicircular section of a diameter of about 2 mm.

The functions of the rings are to facilitate the introduction of the inhaler in the nose and maintain a certain separation between the inner wall of the nasal cavities and the outer wall of the cylindrical tubes, as well as to adjust itself to the nasal cavities in a comfortable and secure way.

The ends of the cylinders that coincide with the external and internal rings are the apertures through which air enters and exits during the phases of inhaling and exhaling.

The exterior average diameter of each cylinder and its corresponding terminal is of 10 mm, although it can vary between 8 and 14 mm, on a scale with intervals of 2 mm.

The size of each terminal is chosen depending on each type of nose in order to facilitate its anatomical adaptability, such as in cases of small noses, prominent ones, etc.

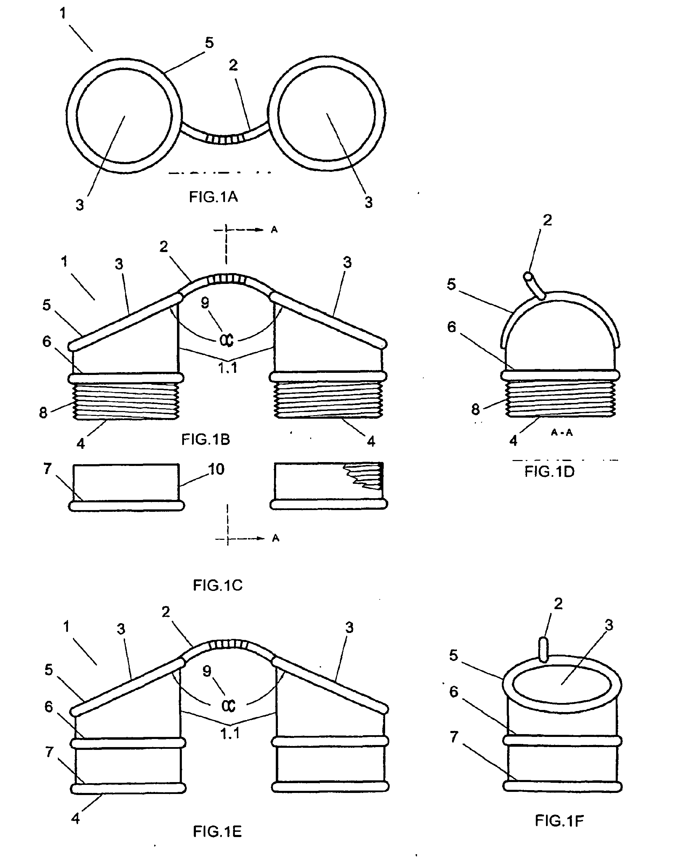

The external planes of aperture of the two cylinders form an angle of 130°, as can be observed in Figure 1B (angle α). In consequence, the contour of each external aperture is elliptical.

The linking bridge between the two cylinders, in the shape of an arch or semicircle, can include a thickening in its centre-front area, with a flexible axis in its interior, for a more personalised adjustment. Said bridge has several functions:
- it acts as a limiting stopper of the depth of penetration of the cylinders into the nasal cavities.
- it also servers as a handle that facilitates both applying and extracting the inhaler.

The appropriate material for producing this mechanism is preferably rigid or semi-rigid plastic, variable in its components.

The inhaler of the invention can feature threaded terminals with a self-blocking system that prevents them from accidentally unscrewing; by way of application with the fingers of a slight pressure on the periphery of the terminals, they are unblocked, thus allowing them to be unscrewed easily.

The objective of this blocking is to provide total security to the user, especially if it is a child, regarding the possibility that the terminal should be loosened within the nose; the possibility of unblocking the screwed-in terminal allows it to be substituted when the use of another one with a different length is required.

### DESCRIPTION OF THE DRAWINGS

In order to complement the description of this invention, and with the objective of improving the understanding of its characteristics, it is accompanied by a series of figures in which, with a purely illustrative and non-limiting purpose, the following pictures are represented, the main components of which are the following.
(1) Inhaler
   (1.1) Cylindrical tube
(2) Bridge
(3) External opening
(4) Internal opening
(5) External ring
(6) Intermediate ring
(7) Internal ring
(8) Threaded area of the cylinder (1.1)
(9) Angle
(10) Short terminal
(11) Long terminal
(12) Male stopper
(13) Female stopper

Figure 1A is a front view of the inhaler (1) (once it has been situated inside the nose).
Figure 1B, is an elevated view of the inhaler (1), with the short terminals (10) unscrewed.
Figure 1C is an elevated view of the short terminals (10) of the inhaler (1).
Figure 1D is the view from the A-A symmetry plane of a section of the inhaler (1) of Figure 1B.
Figure 1E is an elevated view of the inhaler (1) with the short terminals (10) screwed in.
Figure 1F is a lateral profile view of the inhaler (1), with the short terminals (10) screwed in.

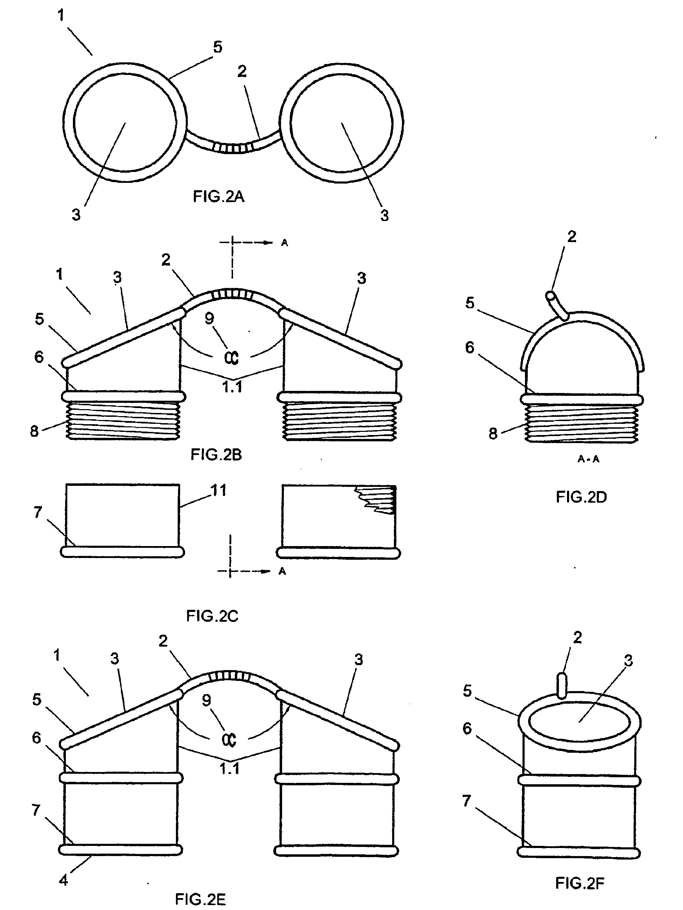

Figure 2A is a front view of the inhaler (1) (once it has been situated inside the nose).
Figure 2B is an elevated view of the inhaler (1), with the long terminals (11) unscrewed.
Figure 2C is an elevated view of the long terminals (11) of the inhaler (1).
Figure 2D is the view from the A-A symmetry plane of a section of the inhaler (1) of Figure 2B.
Figure 2E is the elevated view of the inhaler (1) with the long terminals (11) screwed in.
Figure 2F is a lateral profile view of the inhaler (1) with the long terminals (11) screwed in.
Figure 3A is an elevated view of a type of inhaler (1) **characterised in that** it is provided with a self-blocking system made up of female stoppers (13) and male stoppers (12).
Figure 3B is a section by the A-A plane of Figure 3A, where the male stoppers (12) are shown.
Figure 3C is an elevated view of the threaded terminal, in which the female stopper (13) is detailed.
Figure 3D is a section from the B-B plane of Figure 3C, in which the female stopper (13) is detailed.

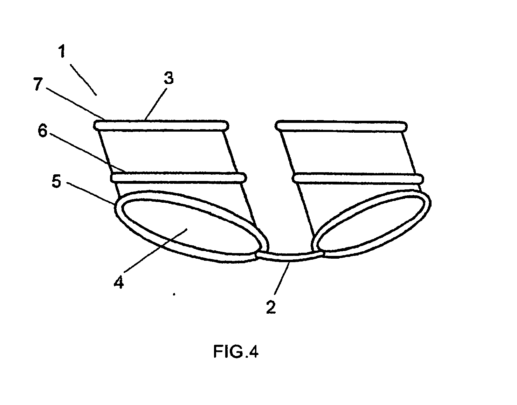

Figure 4 is the view from a perspective of the inhaler drawn in an ideal position for introduction into the nasal cavities.

### PREFERRED EMBODIMENT OF THE INVENTION

Among the different types of anatomical inhalers that can be produced, taking this invention as a basis, the preferred embodiment is the one described below.

This inhaler (1) is characterised by being composed of two cylindrical tubes (1.1), one for each nasal orifice, joined by a linking bridge (2), and two threaded terminals (10, 11). At the end area (8) of each cylinder corresponding to the part that enters more deeply into the nasal cavity, the cylinder is threaded on its exterior. The length of said threaded end area (8) is less than 5 mm, and is preferably 3 mm.

Both cylindrical tubes (1.1) are joined at their anterior or front area by a linking bridge (2) in the shape of an arch or semicircle. This bridge (2) serves as an external stopper on the wall of the nose to prevent the internal progression of the cylinders. At the same time it serves as a handle or holder, for both its application and its extraction.

This bridge (2) or linking component is made up of a strip with a circular section with an equal diameter to that of the external ring (5) of the cylindrical tube (1.1); it may also have a widening at its front-central area, with a flexible axis in its interior for a more personalised fit.

The cylindrical tube (1.1) of each nasal orifice, open at its two ends, is provided with an external opening (3) and with an internal opening (4).

The planes of the external opening (3) form a 130° angle (9).

The external opening (3) of the cylindrical tube (1.1) is provided with an external peripheral ring (5) and, at the area alongside the beginning of the threaded area (8), each cylindrical tube (1.1) is also provided with an intermediate (6), which is also peripheral and constitutes the stopper for the threaded terminal (10, 11). Said threaded terminal, in turn, is provided on its end corresponding to the internal opening (4) of each cylinder, with an internal peripheral ring (7), in order for the inhaler (1) to be adjust in an easy, comfortable and safe way.

All of these rings have a semicircular section with a diameter of about 2 mm.

There are two types of terminals. Short terminals (10) with a length of 5 mm, and long terminals (11) with a length of 9 mm. Both terminals have the same exterior diameter, of 10 mm, in the standard version.

Due to the rounded form that the external (5) and internal (7) rings have, it is very easy to introduce the inhaler into ones nasal cavities in a comfortable and safe way.

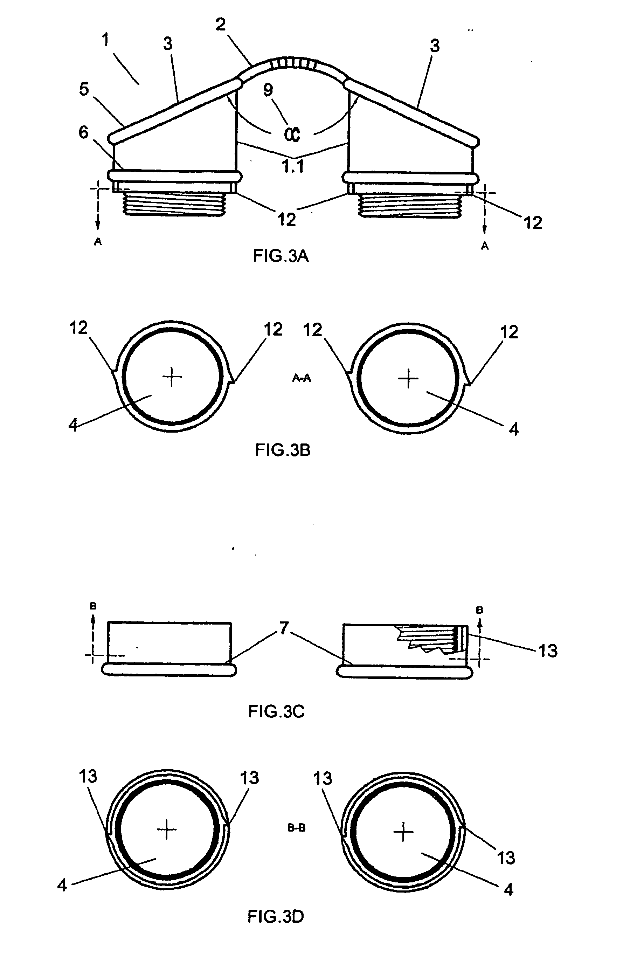

In another preferred embodiment, the threaded terminals (10, 11) are provided with a self-blocking system of a conventional kind that prevents them from accidentally becoming unscrewed; this system can be constituted, among the many existing alternatives for blocking a pair of threaded parts in a reversible way, as indicated in Figures 3A to 3D, by way of shoulders or male stoppers (12) arranged on the periphery of the threaded area (8) of each cylindrical tube (1.1), which, when the terminals (10, 11) are completely threaded, are introduced into notches or female stoppers (13) located at the internal peripheral area of said terminals (10, 11). By applying slight pressure with ones fingers on the periphery of the terminals, they unblock allowing them to be easily unscrewed.

The object of this blockage is to provide total security to the user, especially if it is a child, regarding the possibility that the terminal should be loosened inside their nose; the possibility of unblocking the threaded terminal allows it to be replaced when one of a different length needs to be used.

For all these reasons, the anatomical nasal inhaler can be applied with particular, advantageous features with respect to the previously known solutions.

Having sufficiently described the nature of the present invention, as well as a practical embodiment of the same, it only needs to be added that modifications may be introduced in its shape, size, materials and production procedure, as long as they do not substantially affect the characteristics claimed below.

## Claims

1. Anatomical nasal inhaler for being partially introduced into the nasal cavities in order to facilitate breathing, the inhaler comprising:
- two generally parallel cylindrical tubes (1.1)
- a linking bridge (2) between said cylindrical tubes
**characterised in that** the inhaler additionally comprises
- two cylindrical terminals (10, 11) with threaded interiors
and **in that**
• the interior wall of each cylindrical tube (1.1) is totally smooth;
• each cylindrical tube (1.1) has an end area (8) that is threaded on its exterior for a length of less than 5 mm;
• each cylindrical tube (1.1) is provided with an internal opening (4) with a circular contour and an external opening (3) with an elliptic contour; and
• each of said cylindrical terminals (10, 11) may be screwed on one of said threaded end areas (8) of said cylindrical tubes (1.1).

2. Anatomical nasal inhaler according to claim 1, **characterised in that** each cylindrical tube (1.1) is provided with two peripheral rings: an external one (5) located at the external opening (3) of the cylindrical tube (1.1), and an intermediate one (6) located at the area of said cylindrical tube (1.1) alongside the start of the threaded area (8); and **in that** each terminal (10, 11) is provided at its end corresponding to the internal opening (4) of each cylindrical tube (1.1) with an internal peripheral ring (7) and at its opposite end acts as a stopper against the intermediate ring (6); and in which the section of said peripheral rings (5, 6, 7) is semicircular with a diameter of 2 mm.

3. Anatomical nasal inhaler according to claim 1, **characterised in that** the planes of the external openings (3) of elliptical contours of the cylindrical tubes (1.1) that make up the anatomical nasal inhaler form an angle of 130°.

4. Anatomical nasal inhaler according to claim 1, **characterised** additionally in that at the periphery of the threaded area (8) of each cylindrical tube (1.1) male shoulders or stoppers (12) are located, which, when the terminals (10, 11) are completely screwed in, are introduced into notches or female stoppers (13) located at the internal peripheral area of said terminals (10, 11) to prevent said terminals (10, 11) from being accidentally unscrewed.

5. Anatomical nasal inhaler according to claim 1, **characterised in that** the linking bridge (2) of the cylinders (1.1), in the standard version of the inhaler, is made up of a strip with a circular section of the same diameter as that of the external ring (5).

6. Anatomical nasal inhaler according to claim 1, **characterised in that** the linking bridge (2) of the two cylinders (1.1) in another version of the inhaler, is provided with a widening at its front-central area, with a flexible axis in its interior.

7. Anatomical nasal inhaler according to claim 1, **characterised in that** said threaded terminals (10, 11) have variable height depending on the model of the inhaler.

## Patentansprüche

1. Anatomischer Nasen-Inhalator, der teilweise in die Nasenhöhlen eingeführt wird, um das Atmen zu erleichtern, wobei der Inhalator umfasst:
- zwei im Allgemeinen parallele zylindrische Röhren (1.1),
- eine verbindende Brücke (2) zwischen den zylindrischen Röhren,
**dadurch gekennzeichnet, dass** der Inhalator des Weiteren umfasst:
- zwei zylindrische Endteile (10, 11) mit mit Gewinde versehenen Innenräumen
und dadurch, dass
• die Innenwand jeder zylindrischen Röhre (1.1) vollständig glatt ist;
• jede zylindrische Röhre (1.1) einen Endbereich (8) hat, der an seiner Außenseite über eine Länge von weniger als 5 mm mit Gewinde versehen ist;
• jede zylindrische Röhre (1.1) mit einer Innenöffnung (4) mit einer kreisförmigen Kontur und einer Außenöffnung (3) mit einer elliptischen Kontur versehen ist; und
• jedes der zylindrischen Endteile (10, 11) auf einen der mit Gewinde versehenen Endbereiche (8) der zylindrischen Röhren (1.1) aufgeschraubt werden kann.

2. Anatomischer Nasen-Inhalator nach Anspruch 1, **dadurch gekennzeichnet, dass** jede zylindrische Röhre (1.1) mit zwei Umfangsringen versehen ist, d. h. einem Außenring (5), der sich an der äußeren Öffnung (3) der zylindrischen Röhre (1.1) befindet, und einem Zwischenring (6), der sich an dem Bereich der zylindrischen Röhre (1.1) am Anfang des mit Gewinde versehenen Bereiches (8) befindet, und dass jedes Endteil (10, 11) an seinem der inneren Öffnung (4) jeder zylindrischen Röhre (1.1) entsprechenden Ende mit einem Innen-Umfangsring (7) versehen ist und an seinem gegenüberliegenden Ende als ein Anschlag für den Zwischenring (6) wirkt, und wobei der Querschnitt der Umfangsringe (5, 6, 7) halbkreisförmig mit einem Durchmesser von 2 mm ist.

3. Anatomischer Nasen-Inhalator nach Anspruch 1, **dadurch gekennzeichnet, dass** die Ebenen der äußeren Öffnungen (3) elliptischer Konturen der zylindrischen Röhren (1.1), die den anatomischen Nasen-Inhalator bilden, einen Winkel von 130°bilden.

4. Anatomischer Nasen-Inhalator nach Anspruch 1, zusätzlich **dadurch gekennzeichnet, dass** sich am Umfang des mit Gewinde versehenen Bereiches (8) jeder zylindrischen Röhre (1.1) Außenschultern bzw. Anschläge (12) befinden, die, wenn die Endteile (10, 11) vollständig eingeschraubt sind, in Einkerbungen bzw. Innen-Anschläge (13) eingeführt sind, die sich an dem inneren Umfangsbereich der Endteile (10, 11) befinden, um zu verhindern, dass die Endteile (10, 11) unbeabsichtigt abgeschraubt werden.

5. Anatomischer Nasen-Inhalator nach Anspruch 1, **dadurch gekennzeichnet, dass** die verbindende Brücke (2) der Zylinder (1.1) in der Standardversion des Inhalators aus einem Streifen mit einem kreisförmigen Querschnitt und dem gleichen Durchmesser wie dem des äußeren Rings besteht.

6. Anatomischer Nasen-Inhalator nach Anspruch 1, **dadurch gekennzeichnet, dass** die verbindende Brücke (2) der zwei Zylinder (1.1) in einer anderen Version des Inhalators mit einer Aufweitung an ihrem vorderen mittleren Bereich mit einer flexiblen Achse in ihrem Innenraum versehen ist.

7. Anatomischer Nasen-Inhalator nach Anspruch 1, **dadurch gekennzeichnet, dass** die mit Gewinde versehenen Endteile (10, 11) variable Höhe haben, die vom Modell des Inhalators abhängt.

## Revendications

1. Inhalateur nasal anatomique destiné à être partiellement introduit dans les cavités nasales pour faciliter la respiration, cet inhalateur comprenant :
- deux tubes cylindriques (1.1) essentiellement parallèles,
- un pont de liaison (2) entre ces tubes cylindriques,
**caractérisé en ce que**
l'inhalateur comporte en outre :
- deux embouts d'extrémité cylindriques (10, 11) taraudés à leur partie interne,
et **en ce que**
• la paroi interne de chacun des tubes cylindriques (1.1) est totalement lisse,
• chacun des tubes cylindriques (1.1) a une zone d'extrémité (8) qui est filetée à sa partie externe sur une longueur de moins de 5 mm,
• chacun des tubes cylindriques (1.1) est équipé d'une ouverture interne (4) ayant un contour circulaire et d'une ouverture externe (3) ayant un contour elliptique, et
• chacun des embouts d'extrémité cylindriques (10, 11) peut être vissé sur l'une des zones d'extrémité filetée (8) des tubes cylindriques (1.1).

2. Inhalateur nasal anatomique selon la revendication 1,
**caractérisé en ce que**
chacun des tubes cylindriques (1.1) est équipé de deux anneaux périphériques, à savoir un anneau externe (5) étant situé au niveau de l'ouverture externe (3) du tube cylindrique (1.1) et un anneau intermédiaire (6) situé au niveau de la zone de ce tube cylindrique (1.1) s'étendant le long du début de la zone filetée (8), et
**en ce que** chacun des embouts d'extrémité (10, 11) est équipé d'un anneau périphérique interne (7) à son extrémité correspondant à l'ouverture interne (4) de chacun des tubes cylindriques (1.1) et fait office à son extrémité opposée d'élément d'arrêt venant en butée contre l'anneau intermédiaire (6), la section des anneaux périphériques (5, 6, 7) 7) étant semi circulaire, avec un diamètre de 2 mm.

3. Inhalateur nasal anatomique selon la revendication 1,
**caractérisé en ce que**
les plans des ouvertures externes (3) de contours elliptiques des tubes cylindriques (1.1) qui constituent l'inhalateur nasal anatomique forment un angle de 130°.

4. Inhalateur nasal anatomique selon la revendication 1,
**caractérisé en outre en ce qu'**
il est prévu à la périphérie des zones filetées (8) de chacun des tubes cylindriques (1.1) des épaulements ou éléments d'arrêt males (12), qui, lorsque les embouts d'extrémité (10, 11) sont totalement vissés sont introduits dans des entailles ou éléments d'arrêt femelles (13) situés dans la zone de périphérie interne des embouts d'extrémité (10, 11) pour éviter que ces embouts (10, 11) soient accidentellement dévissés.

5. Inhalateur nasal anatomique selon la revendication 1,
**caractérisé en ce que**
le pont le liaison (2) des cylindres (1.1) est dans le mode de réalisation standard de l'inhalateur, constitué par une bande de section circulaire ayant le même diamètre que l'anneau externe (5).

6. Inhalateur nasal anatomique selon la revendication 1,
**caractérisé en ce que**
le pont de liaison (2) des deux cylindres (1.1) est, dans un autre mode de réalisation de l'inhalateur, équipé dans sa zone frontale centrale, d'un élargissement avec un axe flexible à sa partie interne.

7. Inhalateur nasal anatomique selon la revendication 1,
**caractérisé en ce que**
les embouts d'extrémité taraudés (10, 11) ont une hauteur variable en fonction du modèle d'inhalateur.
